# EUROPÄISCHE PATENTSCHRIFT

(11) **EP 1 699 440 B1**
(45) Veröffentlichungstag und Bekanntmachung des Hinweises auf die Patenterteilung: **11.11.2009**
(21) Anmeldenummer: 04804271.7
(22) Anmeldetag: 23.12.2004
(51) Int. Cl.: A61K 9/20, A61K 31/515, A61K 31/485, A61K 31/5513

(54) **VERFAHREN ZUR HERSTELLUNG EINER GEGEN MISSBRAUCH GESICHERTEN DARREICHUNGSFORM**
METHOD FOR THE PRODUCTION OF AN ADMINISTRATION FORM WHICH IS SECURED AGAINST MISUSE
PROCEDE DE FABRICATION D'UNE FORME D'ADMINISTRATION SECURISEE CONTRE TOUT USAGE DETOURNE

(30) Priorität: 24.12.2003 DE 10361596
(43) Veröffentlichungstag der Anmeldung: 13.09.2006
(73) Patentinhaber: Grünenthal GmbH, 52078 Aachen (DE)
(72) Erfinder: BARTHOLOMÄUS, Johannes, 52080 Aachen (DE); ARKENAU-MARIC, Elisabeth, 50931 Köln (DE)
(74) Vertreter: Bülle, Jan
(86) Internationale Anmeldenummer: PCT/EP2004/014679
(87) Internationale Veröffentlichungsnummer: WO 2005/063214

(56) Entgegenhaltungen:
- US-A1- 2003 068 392
- US-A1- 2003 124 185
- US-B1- 6 309 668

## Beschreibung

Die vorliegende Erfindung betrifft ein Verfahren zur Herstellung von festen, pharmazeutischen Darreichungsformen mit zumindest vermindertem Missbrauchspotential, indem man
a) eine Formulierungsmischung enthaltend wenigstens einen Wirkstoff mit Missbrauchspotential und wenigstens ein synthetisches oder natürliches Polymer (C), das eine Bruchfestigkeit von mindestens 500N aufweist, unter Krafteinwirkung zu Formlingen formt,
b) gegebenenfalls die Formlinge vereinzelt und gegebenenfalls jeweils nach Größen separiert und
c) nach oder während einer Erwärmung bis wenigstens zum Erweichungspunkt des Polymeren (C) solange unter einer Krafteinwirkung lässt, bis die Formlinge eine Bruchhärte von mindestens 500 N aufweisen, gegebenenfalls mit einer Umhüllung versieht und die Formlinge gegebenenfalls alle wieder vermischt.

Eine Vielzahl von pharmazeutischen Wirkstoffen weist neben einer ausgezeichneten Wirksamkeit auf ihrem betreffenden Anwendungsgebiet auch ein Missbrauchspotential auf, d.h. sie können von einem Missbraucher eingesetzt werden, um Wirkungen herbeizuführen, die nicht ihrem Bestimmungszweck entsprechen.
So werden beispielsweise Opiate, die eine exzellente Wirksamkeit bei der Bekämpfung von starken bis sehr starken Schmerzen zeigen, von Missbrauchern häufig zum Einleiten rauschartiger, euphorisierender Zustände verwendet.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Missbraucher gewünschten Ergebnis, nämlich den Kick. Dieser Kick wir auch erreicht, wenn die gepulverte Darreichungsform nasal appliziert, d. h. geschnupft wird.

Da retardierte Darreichungsformen, die Wirkstoffe mit Missbrauchspotential enthalten, üblicherweise selbst bei einer oralen Einnahme von missbräuchlich hohen Mengen nicht zu dem vom Missbraucher gewünschten Kick führen, werden auch diese zum Missbrauch zerkleinert und extrahiert.

Zur Verhinderung des Missbrauchs wurde in dem US-A- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge an Wirkstoff enthält.

Ein entsprechender Ansatz zur Verhinderung des parenteralen Missbrauchs liegt auch der in der WO 95/20947 offenbarten Mehrschichttablette zugrunde, die den Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll der Missbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltexon im Fall von Opiaten, oder Verbindungen, die zu physiologischen Abwehrreaktionen führen, wie z. B. Raolix Ipecacuama = Brechwurz, der Darreichungsform zuzusetzen.

Da aber nach wie vor in den meisten Fällen für den Missbrauch eine Pulverisierung der Darreichungsformen enthaltend einen zum Missbrauch geeigneten Wirkstoff notwendig ist, war es Aufgabe der vorliegenden Erfindung, Verfahren zur Herstellung von gegen Missbrauch geschützten Darreichungsformen zur Verfügung zu stellen, mit denen die dem Missbrauch vorangehende Pulverisierung der Darreichungsform mit den einem potentiellen Missbraucher üblicherweise zur Verfügung stehenden Mitteln erschwert bzw. verhindert und somit eine Darreichungsform für Wirkstoffe mit Missbrauchspotential hergestellt wird, die bei bestimmungsgemäßer Applikation die gewünschte therapeutische Wirkung gewährleistet, aus der aber die Wirkstoffe nicht durch einfaches Pulverisieren in eine zum Missbrauch geeignete Form übergeführt werden können.

Diese Aufgabe wurde durch das erfindungsgemäße Verfahren zur Herstellung von festen, pharmazeutischen Darreichungsformen mit zumindest vermindertem Missbrauchspotential gelöst, das dadurch gekennzeichnet ist, dass man
a) eine Formulierungsmischung enthaltend wenigstens einen Wirkstoff mit Missbrauchspotential, wenigstens ein synthetisches oder natürliches Polymer (C), das eine Bruchfestigkeit von mindestens 500 N aufweist, und ggf. Hilfsstoffe (B) unter Krafteinwirkung zu Formlingen formt,
b) gegebenenfalls die Formlinge vereinzelt und gegebenenfalls jeweils nach Größen separiert und
c) nach oder während einer Erwärmung bis wenigstens zum Erweichungspunkt des Polymeren (C) solange unter einer Krafteinwirkung lässt, bis die Formlinge eine Bruchhärte von mindestens 500 N aufweisen, gegebenenfalls mit einer Umhüllung versieht und die Formlinge gegebenenfalls alle wieder vermischt.

Durch den Einsatz von Polymeren (C) mit der angegebenen Mindestbruchfestigkeit in dem erfindungsgemäßen Verfahren, vorzugsweise in solchen Mengen, dass auch die Darreichungsform eine solche Mindestbruchfestigkeit aufweist, gelingt es, ein Pulverisieren der Darreichungsform mit üblichen Mitteln und damit den anschließenden Missbrauch erheblich zu erschweren bzw. zu verhindern.

Ohne ausreichende Zerkleinerung ist eine parenteral, insbesondere intravenöse, gefahrlose Applikation nicht möglich oder die Extraktion des Wirkstoffes daraus dauert für den Missbraucher zu lange bzw. ein Kick bei missbräuchlicher, oralen Einnahme erfolgt nicht, da keine spontane Freisetzung passiert.

Unter einer Zerkleinerung wird erfindungsgemäß die Pulverisierung der Darreichungsform mit üblichen Mitteln verstanden, die einem Missbraucher üblicherweise zur Verfügung stehen, wie z. B. einen Mörser und Pistill, einen Hammer, ein Schlegel oder andere gebräuchliche Mittel zum Pulverisieren unter Krafteinwirkung, wobei ein gegebenenfalls anfallender Feinanteil (Teilchengröße gleich oder kleiner 0,3 mm) von 5 Gew.% nicht überschritten werden darf.

Die erfindungsgemäß erhaltene Darreichungsform ist daher zur Verhinderung des parenteralen, nasalen und/oder oralen Missbrauchs von pharmazeutischen Wirkstoffen mit Missbrauchspotential geeignet.

Pharmazeutische Wirkstoffe mit Missbrauchspotential sind dem Fachmann ebenso wie deren einzusetzende Mengen und Verfahren zu deren Herstellung bekannt und können als solche, in Form ihrer entsprechenden Derivate, insbesondere Ester oder Ether, oder jeweils in Form entsprechender physiologisch verträglicher Verbindungen, insbesondere in Form ihrer Salze oder Solvate, als Racemate oder Stereoisomere in der erfindungsgemäßen Darreichungsform vorliegen. Die erfindungsgemäß erhaltene Darreichungsform eignet sich auch für die Verabreichung von mehreren Wirkstoffen. Vorzugsweise wird sie zur Verabreichung eines bestimmten Wirkstoffs eingesetzt.

Die erfindungsgemäß erhaltene Darreichungsform eignet sich insbesondere zur Verhinderung des Missbrauchs eines pharmazeutischen Wirkstoffs, der ausgewählt ist aus der Gruppe umfassend Opiate, Opioide, Tranquillantien, vorzugsweise Benzodiazepine, Barbiturate, Stimulantien und weitere Betäubungsmittel.

Ganz besonders eignet sich die erfindungsgemäß erhaltene Darreichungsform zur Verhinderung des Mißbrauchs eines Opiates, Opioids, Tranquillanz oder eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl)}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-a-Methylphenethylamin (Amfetamin), 2-(a-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3H)-on (Bromazepam), 2-Brom-4-(2-chlorphenyl)-9-methyl-6H-thieno[3,2-*f*][1,2,4]triazolo[4,3-a][1,4]diazepin (Brotizolam), 17-Cyclopropylmethyl-4,5a-epopxy-7a[(S)-1-hydroxy-1,2,2-trimethyl-propyl]-6-methoxy-6,14-endo-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*) dion (Clobazam), 5-(2-Chlorphenyl)-7-nitro-1*H*-1,4-benzodiazepin-2(3H)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H* thieno[2,3-e][1,4]diazepin-2(3H)-on (Clotiazepam), 10-Chlor-11b-(2chlorphenyl)-2,3,7,11b-tetrahydrooxazolo[3,2-d][1,4]benzodiazepin-6(5*H*)-on (Cloxazolam), (-)-Methyl-[3β-benzoyloxy-2β(1aH,5a*H*)-tropancarboxylat] (Cocain), 4,5a-Epoxy-3-methoxy-17-methyl-7-morphinen-6a-ol (Codein), 5-(1 -Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5a-Epoxy-3-methoxy-17-methyl-6a-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6aR,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5a-Epoxy-3-ethoxy-17-methyl-7-morphinen-6α-ol (Ethylmorphin), Etonitazen, 4,5a-Epoxy-7α-(1-hydroxy-1-methylbutyl)-6-methoxy-17-methyl-6,14-endo-etheno-morphinan-3-ol (Etorphin), *N-*Ethyl-3-phenyl-8,9,10-trinorboman-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11 b-(2-fluorphenyl)-2,3,7,11b-tetrahydro[1,3]oxazolo[3,2-d][1,4]benzodiazepin-6(5H)-on (Haloxazolam), Heroin, 4,5a-Epoxy-3-methoxy-17-methyl-6-morphinanon (Hydrocodon), 4,5a-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3H)-on (Lormetazepam), 5-(4-Chlorphenyl)-2,5-dihydro-3*H*-imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-a-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5α-Epoxy-17-methyl-7-morphinen-3,6a-diol (Morphin), Myrophin, (±)-*trans*-3-(1,1-Dimethylheptyl)-7,8,10,10a-tetrahydro-1-hydroxy-6,6-dimethyl-6*H*-dibenzo [*b*, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)*-*10-Chlor-2,3,7,11b-tetrahydro-2-methyl-11b-phenyloxazolo[3,2-*d*][1,4] benzodiazepin-6-(5*H*)-on (Oxazolam), 4,5a-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-pheny)-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1H-1,4-benzodiazepin-2(3H)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (11R,2R)-3-(3-Dimethylamino-1-ethyl-2-methyl-propyl)-phenol, (1*R*, 2*R*, 4*S*)-2-[Dimethylamino)methyl-4-(p-fluorbenzyloxy)-1-(m-methoxyphenyl)cyclohexanol, (1 R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäße Darreichungsform eignet sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride.

Weiterhin eignet sich die erfindungsgemäße Darreichungsform insbesondere zur Verhinderung des Missbrauchs eines opioiden Wirkstoffes ausgewählt aus der Gruppe umfassend (1R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1 RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

In dem erfindungsgemäßen Verfahren wird mindestens ein synthetisches oder natürliches Polymeres (C) mit einer Bruchfestigkeit, gemessen nach der in der vorliegenden Anmeldung offenbarten Methode, von mindestens 500 N eingesetzt.

Bevorzugt wird hierfür mindestens ein Polymeres ausgewählt aus der Gruppe umfassend Polyalkylenoxide, vorzugsweise Polymethylenoxide, Polyethylenoxide, Polypropylenoxide, Polyethylene, Polypropylene, Polyvinylchloride, Polycarbonate, Polystyrrole, Polyacrylate, deren Copolymerisate, und Mischungen aus mindestens zwei der genannten Polymeren eingesetzt. Die Polymeren zeichnen sich durch ein Molekulargewicht von mindestens 0,5 Millionen, bestimmt durch rheologische Messung, aus. Ganz besonders bevorzugt sind thermoplastische Polyalkylenoxide, wie Polyethylenoxide, mit einem Molekulargewicht von mindestens 0,5 Mio., vorzugsweise mindestens 1 Mio. bis 15 Mio., bestimmt durch rheologische Messung. Diese Polymeren weisen eine Viskosität bei 25°C von 4500 bis 17600 cP, gemessen an einer 5 Gew.% wässrigen Lösung mit Hilfe eines Brookfield Viskosimeter, Model RVF (Spindel Nr. 2 / Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 / Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden vorzugsweise als Pulver eingesetzt. Sie können in Wasser löslich sein.

In der Formulierungsmischung bzw. in den erfindungsgemäß hergestellten Darreichungsformen sind mindestens 30 Gew.%, vorzugsweise mindestens 50 Gew.% bis 99,9 Gew.%, bezogen auf die Gesamtmenge, Polymere (C) vorhanden.

### [EA1]

Des weiteren können zusätzlich zur Erzielung der notwendigen Bruchfestigkeit der erfindungsgemäß erhaltene Darreichungsform mindestens ein natürliches oder synthetisches Wachs (D) mit einer Bruchfestigkeit, gemessen nach der in der vorliegenden Anmeldung offenbarten Methode von mindestens 500 N mitverwendet werden.

Bevorzugt werden Wachse mit einem Erweichungspunkt von mindestens 60°C eingesetzt. Besonders bevorzugt sind Carnaubawachs und Bienenwachs. Ganz besonders bevorzugt ist Carnaubawachs. Carnaubawachs ist ein natürliches Wachs, das aus den Blättern der Camaubapalme gewonnen wird und einen Erweichungspunkt von wenigstens 80°C aufweist. Beim zusätzlichen Einsatz der Wachskomponente (D) wird diese zusammen mit wenigstens einem Polymeren (C) in solchen Mengen eingesetzt, dass die Darreichungsform eine Bruchfestigkeit von mindestens 500 N aufweist.

Als Hilfsstoffe (B) können die üblichen für die Formulierung von festen Darreichungsformen bekannten Hilfsstoffe verwendet werden. Vorzugsweise sind dies Weichmacher, wie Polyethylenglykole, Hilfsstoffe, die Wirkstofffreisetzung beeinflussend, vorzugsweise hydrophobe oder hydrophile, vorzugsweise hydrophile Polymere, ganz besonders bevorzugt Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Zur Herstellung der gegen Missbrauch gesicherten, festen Darreichungsform wird der Wirkstoff, die Komponente (C), ggf. die Wachskomponente (D), ggf. Hilfsstoffe (B) und ggf. mindestens eine der nachfolgend aufgeführten ggf. vorhandenen weiteren missbrauchsverhindernden Komponenten (a) - (f) zunächst vermischt und die resultierende Formulierungsmischung durch Krafteinwirkung zu Formlingen, vorzugsweise zu der Darreichungsform, geformt.

Die Bereitstellung der Formulierungsmischung erfolgt in einem dem Fachmann bekannten Mischgrät. Das Mischgerät kann beispielsweise ein Wälzmischer, Schüttelmischer, Schermischer oder Zwangsmischer sein.

Die resultierende Formulierungsmischung wird vorzugsweise direkt durch Krafteinwirkung zu Formlingen, vorzugsweise zu der Darreichungsform, vorzugsweise ohne Wärmeeinwirkung, geformt. Beispielsweise kann die Formulierungsmischung durch Direkttablettierung zu Tabletten geformt werden. Bei einer Direkttablettierung wird mit Hilfe eines Tablettierwerkzeuges, d. h. einem Unterstempel, Oberstempel und einer Matrize verpresst.

Die Formulierungsmischung kann auch zuerst granuliert und anschließend geformt werden.

Die Formgebung wird vorzugsweise unter einer Krafteinwirkung durchgeführt, wobei eine Kraft von gleich oder größer 0,5 kN, vorzugsweise von 1 bis 100 kN, angewendet wird. Die Kraft wird vorzugsweise mit Hilfe einer Presse, vorzugsweise Tablettenpresse, mit Formwalzen oder mit Walzen ausgerüstete Formbänder ausgeübt. Die Formulierungsmischung kann auch mit Hilfe eines Extruders zu einem Strang extrudiert werden, der zu Formlingen mit der gewünschten Größe vereinzelt wird. Wenn bei der Krafteinwirkung auch eine Erwärmung erfolgt, sollte die Erwärmung unter 60°C bleiben.

Sofern die Formulierungsmischung zu multipartikulären Formlingen, wie Granulaten, Pellets, verarbeitet wird, sollten diese eine Mindestgröße von 0,5 mm, vorzugsweise eine Größe von 1 bis 3,5 mm aufweisen. Vor der Weiterverarbeitung können diese Formlinge, soweit sie nicht eine weitgehend einheitliche Größe aufweisen, vorzugsweise nach Größen separiert werden. Diese Separierung kann mit Hilfe der Siebtechnik erfolgen.

Die Formlinge werden in dem weiteren Verfahrensschritt c) nochmals einer Krafteinwirkung ausgesetzt, wobei entweder vor oder während der Krafteinwirkung die Formlinge bis wenigstens zum Erweichungspunkt des Polymeren (C), vorzugsweise auf gleich oder größer 60°C erwärmt werden. Es wird eine Kraft von mindestens 0,1 kN, vorzugsweise von 1 kN bis zu 120 kN, besonders bevorzugt bis zu 100 kN, ganz besonders bevorzugt bis maximal 90 kN, angewandt. Die Dauer der Behandlung mit Kraft ist, wie für jeden Fachmann bekannt, von der Stärke der angewandten Kraft, der Erwärmung vor oder während der Krafteinwirkung und ggf. von der Größe der Formlinge abhängig und kann durch einfache Versuche ermittelt werden, damit die Formlinge nach der Krafteinwirkung eine Bruchhärte von mindestens 500 N, gemessen nach der nachfolgend angegebenen Methode, aufweisen.

Die notwendige Erwärmung kann vorzugsweise durch eine Temperaturmessung im Inneren eines Formlings mit Hilfe eines Temperaturfühlers kontrolliert werden.

Die Krafteinwirkung kann mit Hilfe der vorstehend angegebenen Apparaturen kontinuierlich oder diskontinuierlich erfolgen. Das gesamte erfindungsgemäß0e Verfahren kann kontinuierlich und diskontinuierlich erfolgen.

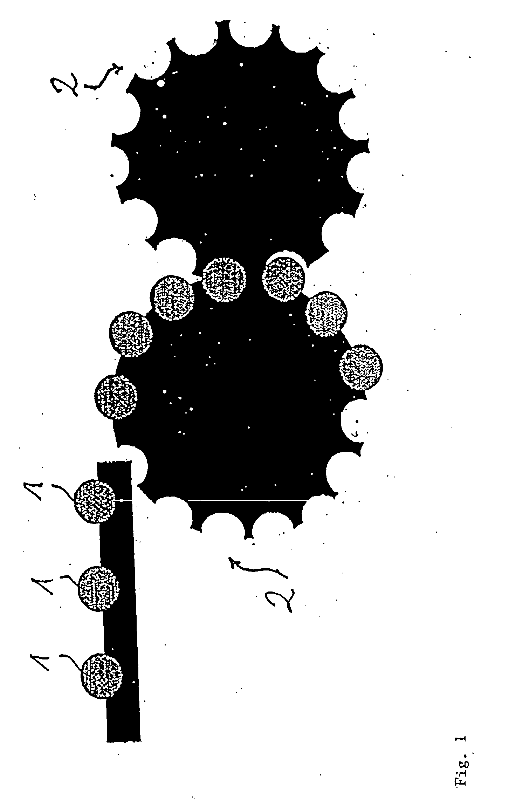

Figur 1 zeigt eine Vorrichtung, mit der die Formlinge 1, hier Tabletten, nach der Erwärmung zwischen Formbändern mit Druckwalzen 2 eine Krafteinwirkung erfahren. Dabei sind die parallel oben und unten laufenden Formbänder mit Mitteln zur Aufnahme der Tabletten ausgerüstet. Mit den Formbändern kann jeweils auch eine Folie, vorzugsweise eine Aluminiumfolie oder eine funktionelle Folie, mitlaufen - in Figur 1 nicht abgebildet -, so dass während der Krafteinwirkung auch gleichzeitig eine Umhüllung des Formlings 1, hier Tablette, erfolgen kann. So umhüllte Formlinge können zu einer gewünschten Anzahl von zusammenhängenden Darreichungsformen, wie z. B. Blistern, vereinzelt werden.

Die Erwärmung der Formlinge kann auf verschiedenste Art und Weise durchgeführt werden. Bevorzugt ist ein Erhitzen in Öfen, d. h. mit Hilfe einer erwärmten Gasatmosphäre, oder mit Strahlungswärme. Es ist auch möglich, die Erwärmung durch elektromagnetische Wellen, insbesondere durch Mikrowellen, durchzuführen. Neben Öfen, die im diskontinuierlichen Betrieb befüllt werden, sind auch Tunnelöfen geeignet, in denen die Formlinge kontinuierlich durch diesen Ofen transportiert werden. In einem weiteren bevorzugten Verfahrensvariante erfolgt die Wärmezufuhr über das Transportband zu den Formlingen (1).
Die Erwärmung erfolgt vorzugsweise unter einer Schutzgasatmosphäre, besonders bevorzugt unter Stickstoffatmosphäre.

Wie bereits ausgeführt, kann die Krafteinwirkung mit Hilfe einer Tablettenpresse erfolgen, wobei die Formlinge der Matrize erwärmt zugeführt werden. Insbesondere kann dies auch mit einer Manteltablettenherstellung gekoppelt werden, wobei das äußere Hüllmaterial, das aufgepresst wird, aus Hilfsstoffen oder aus einer Wirkstoff/Hilfsstoffmischung bestehen kann.

Besonders bevorzugt ist eine Verfahrensweise, bei der die Krafteinwirkung gemäß c) über Formwalzen erfolgt (s. Figur 1). Bei dieser Verfahrensweise werden die erwärmten Formlinge (1) zwei gegenläufigen Druckwalzen (2) zugeführt, die als Profil-Vertiefungen zur Aufnahme der einzelnen Tabletten aufweisen. Zwischen den Walzen führt die Krafteinwirkung auf die erwärmten Formlinge (1) zur gewünschten Bruchfestigkeit der Darreichungsform.

Diese Verfahrensweise eignet sich auch für eine kontinuierliche Durchführung, wobei die Zufuhr der Formlinge zu den Walzen über ein Transportband erfolgt, über das die Formlinge zuvor direkt der Erwärmung im Tunnelofen, unter einer Strahlungsquelle oder über das Band zugeführt werden, bevor sie der Krafteinwirkung ausgesetzt werden.

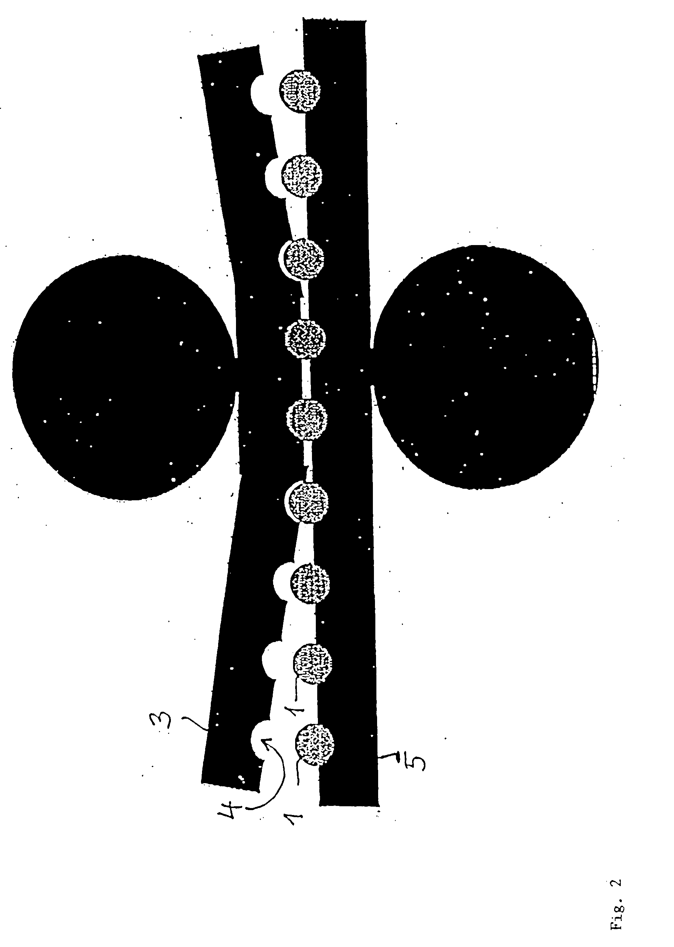

In einer weiteren bevorzugten Arbeitsweise werden die Formlinge (1) in einem Träger (3), der ein Profil für die Formlinge (1) aufweist und besonders bevorzugt als Endlos- Transportband ausgelegt ist, transportiert. Dieser Träger (3) wird mit einem zweiten Formband (5), das ebenso ein Teilprofil der Formlinge (1) aufweist, in Deckung gebracht und auf beide Seiten der Trägerbänder Kraft ausgeübt. Diese Verfahrensweise wird in **Figur 2** dargestellt.

In dem erfindungsgemäßen Verfahren kann es von Vorteil sein, auf den Formprofilen, in denen auf die Formlinge Kraft ausgeübt wird, und auf die Formlinge Trennmittel aufzubringen, damit die Formlinge wieder leicht vom Trägerband bzw. den Druckwalzen abgelöst werden können. Als Trennmittel eignen sich pharmazeutisch übliche Trennmittel, wie z. B. Talkum, Magnesiumstearat. Bevorzugt sind Trennmittel, die bei der Prozesstemperatur ihren Aggregatzustand nicht ändern.

Weiterhin kann es vorteilhaft sein, in den Vorrichtungen, mit deren Hilfe die Krafteinwirkung ausgeübt wird, mechanische Trennhilfen vorzusehen, die die Formlinge nach der Krafteinwirkung aktiv ausstoßen. Dies kann z. B. durch Löcher, durch die unter Druck ein Gas geblasen wird, erfolgen oder durch mechanische Stempel.

Zur Beschleunigung und Optimierung des erfindungsgemäßen Verfahrens können die Formlinge nach der Krafteinwirkung gemäß c) rasch abgekühlt werden. Dies kann z. B. durch Transport der Formlinge in oder durch einen Kühlraum erfolgen oder durch Einbringen in ein Kühlmedium, wie z. B. in ein flüssiges Gas.

Die erfindungsgemäß erhaltenen Darreichungsformen zeichnen sich dadurch aus, dass sie aufgrund ihrer Härte selbst Abkühlung auf tiefe Temperaturen nicht zu pulverisieren, z. B. durch Mörsern, sind. Ein oraler, parenteraler, inbesondere intravenöser oder nasaler Missbrauch ist dadurch praktisch ausgeschlossen.

Um jedoch jeden möglichen Missbrauch bei einer Zerkleinerung und /oder bei einer dennoch ggf. durch außergewöhnliche Krafteinwirkung auftretende Pulverisierung der erfindungsgemäß erhaltenen Darreichungsformen vorzubeugen, können die erfindungsgemäß erhaltenen Darreichungsformen in einer bevorzugten Ausführungsform als Hilfsstoffe (B) weitere missbrauchs-erschwerende bzw. - verhindernde Mittel enthalten.

So kann die erfindungsgemäß erhaltene, gegen Missbrauch gesicherte Darreichungsform, die neben einem oder mehreren Wirkstoffen mit Missbrauchspotential, mindestens einem härtebildenden Polymer (C), ggf. Hilfsstoffe (B) und ggf. mindestens einen Wachs (D) noch wenigstens eine der nachfolgenden Komponenten (a)-(e) als ggf. weitere Hilfsstoffe (B) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum.
(e) wenigstens einen Farbstoff als aversives Mittel
(f) wenigstens einen Bitterstoff

Die Komponenten (a) bis (f) sind jeweils für sich allein zusätzlich zur Sicherung der erfindungsgemäßen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen, Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten, gelingt es, bei erfindungsgemäß erhaltenen Darreichungsformen noch effektiver der Missbrauch zu erschweren.

In einer Ausführungsform kann die erfindungsgemäße Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die erfindungsgemäß erhaltene Darreichungsform sämtliche Komponenten (a)-(f) aufweisen.

Sofern die erfindungsgemäß erhaltene Darreichungsform gegen Missbrauch die Komponente (a) umfasst, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass der die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so dass der Missbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Unter Darreichungseinheit wird eine separate bzw. separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, β-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäß erhaltene Darreichungsform die Pflanzenteile der entsprechenden Scharfstoffdrogen in einer Menge von 0,01 bis 30 Gew.%, besonders bevorzugt 0,1 bis 0,5 Gew.%, jeweils bezogen auf das Gesamtgewicht der Darreichungseinheit, enthalten.
Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.%, bezogen auf das Gesamtgewicht der Darreichungseinheit.

Eine weitere Möglichkeit bei der erfindungsgemäß erhaltenen Darreichungsform gegen Missbrauch vorzubeugen, besteht darin, wenigstens ein viskositätserhöhendes Mittel als weitere Missbrauchs-verhindernde Komponente (b) der Darreichungsform zuzusetzen, das in einem mit Hilfe einer notwendigen Mindestmenge an einer wässrigen Flüssigkeit aus der Darreichungsform gewonnenen Extrakt ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, wirkstoffhaltige Gel beim Einbringen vorzugsweise mit Hilfe einer Injektionsnadel, in eine weitere Menge wässriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wässriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. In Kombination mit mindestens einer ggf. vorhandenen Komponente (a) bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schäden des Missbrauchers führen.

Zur Überprüfung, ob ein viskositätserhöhendes Mittel als Komponente (b) zur Anwendung in der erfindungsgemäß erhaltenen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur Missbrauchs-Vorbeugung bzw. - Verhinderung bei den erfindungsgemäßen Darreichungsformen.

Sofern der erfindungsgemäß erhaltenen Darreichungsform die Komponente (b) hinzugefügt wird, kommen vorzugsweise eine oder mehrere viskositätserhöhende Mittel zum Einsatz, die ausgewählt sind aus der Gruppe bestehend aus mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5°, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine, vorzugsweise aus Citrusfrüchten oder Äpfeln, (Cesapectin^{®} HM Medium Rapid Set), Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakernmehl (Polygum 43/1^{®}), Propylenglykoalginat (Protanal-Ester SD-LB^{®}),,
Natrium-Hyaluronat, Tragant, Taragummi (Vidogum SP 200^{®}), fermentiertes Polysaccharid- Welan Gum (K1A96), Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist eine Menge von 0,1 bis 20 Gew.%, besonders bevorzugt von 0,1 bis 15 Gew.%, bezogen auf das Gesamtgewicht der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von gleich oder größer 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wässriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge einer wässrigen Flüssigkeit, ein Gel bildet.

Es ist auch möglich, die viskositätserhöhenden Mittel und die übrigen Bestandteile in räumlich voneinander getrennter Anordnung in der erfindungsgemäß erhaltenen Darreichungsform zu formulieren.

Des weiteren kann die erfindungsgemäß erhaltene Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäß erhaltenen Darreichungsform vorliegen und keine Wirkung bei bestimmungsgemäßer Verwendung entfalten.

Geeignete Antagonisten zur Verhinderung des Missbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäß erhaltenen Darreichungsform vorliegen.

Sofern der in der Darreichungsform vorliegende Wirkstoff ein Opiat oder ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von gleich oder größer 1 mg, besonders bevorzugt in einer Menge von 3 bis 100 mg, ganz besonders bevorzugt in einer Menge von 5 bis 50 mg auf pro Darreichungsform, d. h. pro Dosiereinheit eingesetzt.

Weist die erfindungsgemäß erhaltene Darreichungsform als Wirkstoff ein Stimulanz auf, ist der Antagonist bevorzugt ein Neuroleptikum, vorzugsweise wenigstens eine Verbindung ausgewählt aus der Gruppe bestehend aus Haloperidol, Promethacin, Fluophenozin, Perphenazin, Levomepromazin, Thioridazin, Perazin, Chlorpromazin, Chlorprotheaxin, Zucklopantexol, Flupentexol, Prithipendyl, Zotepin, Penperidol, Piparmeron, Melperol und Bromperidol.

Vorzugsweise weist die erfindungsgemäß erhaltene Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur Vorbeugung und Sicherung der erfindungsgemäß erhaltenen Darreichungsform gegen Missbrauch die Komponente (d) umfasst, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemäßer Anwendung keine Wirkung im Körper entfalten sollte.

Geeignete Emetika zur Verhinderung des Missbrauchs eines Wirkstoffs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäß erhaltenen Darreichungsform vorliegen.

In der erfindungsgemäß erhaltenen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäß erhaltene Darreichungsform als Komponente (d) das Emetikum Emetin aufweisen, bevorzugt in einer Menge von gleich oder größer 3 mg, besonders bevorzugt gleich oder größer 10 mg und ganz besonders bevorzugt in einer Menge von gleich oder größer 20 mg pro Darreichungsform, d. h. Dosiereinheit.

Ebenfalls bevorzugt kann als Emetikum Apimorphin in der erfindungsgemäßen Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiereinheit.

Sofern die erfindungsgemäß erhaltene Darreichungsform die Komponente (e) als weiteren missbrauchsverhindernden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäß erhaltene Darreichungsform als weiteren missbrauchsverhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat. Besonders bevorzugt ist Denatonium-Benzoat.

Die erfindungsgemäß erhaltene feste Darreichungsform eignet sich zur oralen, vaginalen oder rektalen, vorzugsweise zur oralen Einnahme bei Mensch und Tier. Vorzugsweise ist sie nicht filmförmig. Die oral applizierbare, erfindungsgemäße Darreichungsform kann in multipartikulärer Form, bevorzugt in Form von Mikrotabletten, Mikrokapseln, Mikropellets, Granulaten, Sphäroiden, Perlen oder Pellets, ggf. in Kapseln abgefüllt oder zu Tabletten verpresst, vorliegen. Vorzugsweise weisen die multipartikulären Formen einer Mindestgröße von 0,5 mm, besonders bevorzugt im Bereich von 1 bis 3,5 mm, auf. Je nach gewünschter Darreichungsform werden ggf. auch die üblichen Hilfsstoffe (B) zur Formulierung der Darreichungsform mitverwendet.

In einer weiteren bevorzugten Ausführungsform liegt die erfindungsgemäße Darreichungsform in Form einer Tablette, einer Kapsel oder in Form eines oralen osmotischen therapeutischen Systems (OROS) vor, vorzugsweise wenn mindestens noch eine weitere missbrauchsverhindernde Komponente (a) - (f) vorhanden ist.

Sofern die Komponenten (c) und/oder (d) und/oder (f) in der erfindungsgemäß hergestellte Darreichungsform vorhanden sind, ist darauf zu achten, dass sie so formuliert oder so gering dosiert sind, dass sie bei bestimmungsgemäßer Applikation der Darreichungsform praktisch keine den Patienten oder die Wirksamkeit des Wirkstoffs beeinträchtigende Wirkung entfalten können.

Sofern die erfindungsgemäß hergestellte Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung der Darreichungsform versehentlich, insbesondere durch Kinder, oder beim Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäß hergestellten Darreichungsform zur Sicherung der Darreichungsform enthaltend die Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen den (die) Wirkstoff(e), vorzugsweise auch mindestens ein Polymer (C) und die ggf. vorhandene Komponente (D) und gegebenenfalls wenigstens eine der gegebenenfalls vorhandenen Komponenten (a) und/oder (b) und/oder (e) bzw. vorzugsweise jeweils wenigstens ein Polymer (C) und ggf. (D) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass vorzugsweise jede der Untereinheiten nach dem vorstehend angegebenen, erfindungsgemäßen Verfahren formuliert werden, sofern die mechanische Fertigkeit gewünscht bzw. erforderlich ist.

Ein wesentlicher Vorteil der getrennten Formulierung der Wirkstoffe von den Komponenten (c) bzw. (d) bzw. (f) in Untereinheiten (X) und (Y) der erfindungsgemäß hergestellten Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, wenn erforderlich zur Missbrauchsverhinderung, dass die jeweiligen Untereinheiten das Polymer (C) enthalten und in der angegebenen Weise formuliert und erfindungsgemäß hergestellt wurden.

Sollte es den Missbrauchem wider Erwarten gelingen, eine solche erfindungsgemäße Darreichungsform, welche die Komponenten (c) und/oder (e) und/oder (d) und/oder (f) in Untereinheiten (Y) aufweist, zum Zwecke der missbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer erfindungsgemäßen Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäß hergestellten Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) vorzugsweise mit formuliert und die Formulierung vorzugsweise gemäß dem erfindungsgemäßen Verfahren durchgeführt wird.

In einer bevorzugten Ausführungsform der erfindungsgemäß hergestellten Darreichungsform liegen die Untereinheiten (X) und (Y) in multipartikulärer Form vor, wobei Granulate, Sphäroide, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y) durch mechanische Auslese möglich ist. Die multipartikulären Formen weisen bevorzugt eine Größe im Bereich von 0,5 bis 3,5 mm, vorzugsweise 0,5 bis 2 mm auf.

Die Untereinheiten (X) und (Y) in multpartikulärer Form können auch bevorzugt in eine Kapsel abgefüllt oder zu einer Tablette verpresst werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

Die multipartikulären Untereinheiten können auch als Slurry oder als Suspension in pharmazeutisch unbedenklichen Suspensionsmedien als orale Darreichungsform formuliert werden.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäß hergestellten Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so dass neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäß hergestellte Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die vorzugsweise der erfindungsgemäßen Härteanforderung genügen sollte, in ein- und derselben multipartikulären Form nach dem erfindungsgemäßen Verfahren formuliert werden.

In einer weiteren bevorzugten Ausführungsform der erfindungsgemäß hergestellten Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäß hergestellten Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäß hergestellte Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette, eines Coextrudats oder Laminats vor, das nach dem erfindungsgemäßen Verfahren hergestellt wurde.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') sollte vorzugsweise die erfindungsgemäßen Härtevoraussetzungen erfüllen.

Ebenfalls besonders bevorzugt ist eine Ausführungsform der erfindungsgemäß hergestellten Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

In einer weiteren bevorzugten Ausführungsform hat die Untereinheit (X) der erfindungsgemäß hergestellten Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der erfindungsgemäßen Herstellung der jeweiligen Darreichungsformen jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäß hergestellten Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und ggf. (D) enthalten und vorzugsweise erfindungsgemäß hergestellt worden sind.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.
Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe umfassend Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäß hergestellte Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäß hergestellte Darreichungsform weist eine kontrollierte Freisetzung des Wirkstoffes auf. Sie eignet sich dabei vorzugsweise für eine mehrmalige tägliche Verabreichung an Patienten, wie z. B. zur Schmerzbekämpfung bei menschlichen Patienten.

Die erfindungsgemäß hergestellte Darreichungsform kann einen oder mehrere Wirkstoffe zumindest teilweise in einer darüber hinaus retardierter Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, dass die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf. vorhandenen Komponente (c) und/oder (d) eine beeinträchtigende Wirkung entfalten können.

Außerdem darf durch die Zugabe von retardierenden Materialien keine Beeinträchtigung der notwendigen Härte erfolgen.

Die kontrollierte Freisetzung aus der erfindungsgemäß hergestellten Darreichungsform wird vorzugsweise durch Einbettung des Wirkstoffes in eine Matrix erzielt. Die als Matrixmaterialien dienenden Hilfsstoffe kontrollieren die Wirkstofffreisetzung. Matrixmaterialien können beispielweise hydrophile, gelbildende Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch die Komponenten (C) und ggf. vorhandene Komponente (D), die zur Erzielung der erfindungsgemäß erhaltenen Bruchfestigkeit von mindestens 500 N dienen, als zusätzliche Matrixmaterialien wirken.

Sofern die erfindungsgemäß hergestellte Darreichungsform zur oralen Applikation vorgesehen ist, kann sie bevorzugt auch einen magensaftresistenten Übe rzug aufweisen, der sich in Abhängigkeit vom pH-Wert der Freisetzungsumgebung auflöst.

Durch diesen Überzug kann erreicht werden, dass die erfindungsgemäß hergestellte Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturi ng Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

### Methode zur Bestimmung der Bruchfestigkeit

A) Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Materials, bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäisches Arzneibuch 1997, Seite 143, 144, Methode Nr. 2.9.8. unter Einsatz der nachstehend aufgeführten Apparatur die Bruchfestigkeit bestimmt. Als Apparatur für die Messung wird ein Einsäulen-Tischmodell mit der Bezeichnung "Material-Prüfmaschine TMTC-FR2.5 TH.D09" der Firma Zwick GmbH & Co. KG, Ulm, Deuschland, Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

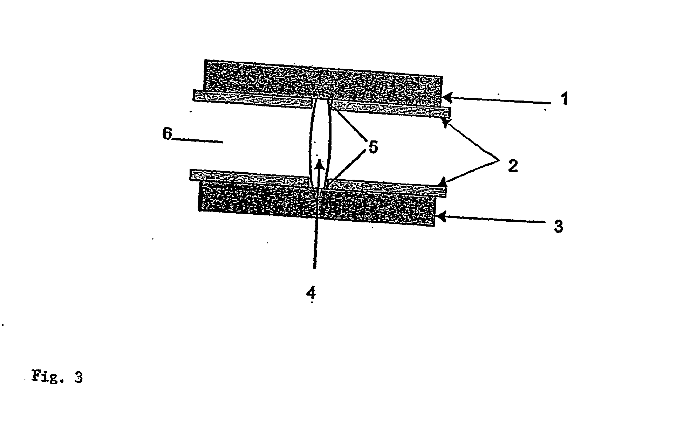

   Figur 3 zeigt die Messung der Bruchfestigkeit einer Tablette, insbesondere die dafür eingesetzte Justierungsvorrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.
   Als bruchfest bei einer bestimmten Krafteinwirkung werden auch die Tabletten eingestuft, bei denen kein Bruch feststellbar, aber ggf. eine plastische Verformung der Tablette durch die Krafteinwirkung erfolgt ist.
   Bei den erfindungsgemäß hergestellten Darreichungsformen, wird die Bruchfestigkeit nach der aufgeführten Meßmethode bestimmt, wobei von Tabletten abweichende Darreichungsformen ebenso geprüft werden.

Im folgenden wird die Erfindung anhand von Beispielen erläutert. Diese Erläuterungen sind lediglich beispielhaft und schränken den allgemeinen Erfindungsgedanken nicht ein.

### Beispiele:

In einer Reihe von Beispielen wurde Tramadolhydrochlorid als Wirkstoff verwendet. Tramadolhydrochlorid wurde verwendet, obwohl Tramadol kein Wirkstoff mit üblichem Mißbrauchspotential ist und daher nicht unter das Betäubungsmittelgesetz fällt, weil dadurch das experimentelle Arbeiten erleichtert wird. Tramadol ist außerdem ein Vertreter der Klasse der Opioide mit ausgezeichneter Wasserlöslichkeit.

### Beispiel 1:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 7 000 000 (Polyox WSR 303, Dow Chemicals) | 221,0 mg | 132,6 g |
| Hydroxypropylmethylcellulose 100 000 cP | 20,0 mg | 12,0 g |
| (Metholose 90 SH 100 000) | | |
| Magnesium-Stearat | 9,0 mg | 5,4 g |
| Gesamtgewicht | 350,0 mg | 210,0 g |

Tramadol Hydrochlorid, Polyethylenoxidpulver und Hydroxypropylmethylcellulose wurden in einem Freifallmischer gemischt. Anschließend wurde das Magnesiumstearat-Pulver zugemischt. Die Pulvermischung wurde auf einer Ekzenterpresse vom Typ Korsch EK0 zu Tabletten gepresst. Das Tablettierwerkzeug hat einen Durchmesser von 10 mm und einen Wölbungsradius von 8 mm. Diese Tabletten wurden mit Hilfe eines Labor-Siegelgeräts (Kopp Labor-Siegelgerät SPGE 20 Siegelnahtfestigkeitsprüfgerät Hot- und COLD-Tack der Fa. Kopp) weiterbehandelt. Anstelle der Siegelschienen wurden zwei Metallschienen eingebaut. In diese wurde jeweils eine konkave Wölbung eingefräst, die einen Durchmesser von 10 mm und einen Radius von 8 mm aufweist. Die Oberfläche der Wölbung ist mit Teflon beschichtet. Nach Einbau der Schienen in das Siegelgerät ergeben zwei komplementäre Wölbungen eine Linsenform, in die die Tabletten jeweils eingelegt wurden.
Die Siegelbacken wurden zuvor auf 130°C aufgeheizt, die Tabletten eingelegt und darauf eine Kraft von 750 N für 2,5 min ausgeübt.

Die Bruchfestigkeit der Tabletten wird nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

Die in vitro Freisetzung des Wirkstoffs Tramadol aus den Tabletten wurde in einer Blattrührerapparatur mit Sinker nach Pharm Eur. bestimmt. Die Temperatur des Freisetzungsmediums betrug 37°C und die Umdrehungsgeschwindigkeit des Rührers 75min⁻¹. Als Freisetzungsmedium wurde ein Darmsaft mit pH6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 21 % |
| 240 min | 70 % |
| 480 min | 94 % |
| 720 min | 100 % |

### Beispiel 2:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 5 000 000 (Polyox WSR Coagulant, Dow Chemicals) | 221,0 mg | 132,6 g |
| Hydroxypropylmethylcellulose 100 000 cP (Metholose 90 SH 100 000) | 20,0 mg | 12,0 g |
| Magnesium-Stearat | 9,0 mg | 5,4 g |
| Gesamtgewicht | 350,0 mg | 210,0 g |

Wie in Beispiel 1 angegeben, wurden Tabletten mit einem Durchmesser von 10 mm und einem Wölbungsradius von 8 mm hergestellt.

Auch die Weiterbehandlung der Tabletten erfolgte wie im Beispiel 1 mit dem Unterschied, dass die Siegelbacken auf 100°C aufgeheizt wurden.

Die Bruchfestigkeit der Tabletten werden nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37°C und die Umdrehungsgeschwindigkeit des Rührers 75min⁻¹. Als Freisetzungsmedium wurde ein Darmsaft mit pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 17 % |
| 240 min | 60 % |
| 480 min | 84 % |
| 720 min | 95 % |

### Beispiel 3:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 7 000 000, Feinpulver (Polyox WSR 303 FP, Dow Chemicals) | 221,0 mg | 132,6 g |
| Hydroxypropylmethylcellulose 100 000 cP (Metholose 90 SH 100 000) | 20,0 mg | 12,0 g |
| Magnesium-Stearat | 9,0 mg | 5,4 g |
| Gesamtgewicht | 350,0 mg | 210,0 g |

Es wurden wie in Beispiel 1 beschrieben Tabletten hergestellt. Auch die Weiterbehandlung der Tabletten erfolgte wie in Beispiel 1 aufgeführt.

Die Bruchfestigkeit dieser Tabletten wurde nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37°C und die Umdrehungsgeschwindigkeit des Rührers 75min⁻¹. Als Freisetzungsmedium wurde ein Darmsaft mit pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 21 % |
| 240 min | 69 % |
| 480 min | 93 % |
| 720 min | 100 % |

### Beispiel 4:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 7 000 000 (Polyox WSR 303, Dow Chemicals) | 221,0 mg | 132,6 g |
| Hydroxypropylmethylcellulose 100 000 cP (Metholose 90 SH 100 000) | 20,0 mg | 12,0 g |
| Magnesium-Stearat | 9,0 mg | 5,4 g |
| Gesamtgewicht | 350,0 mg | 210,0 g |

Wie in Beispiel 1 angegeben, wurden Tabletten hergestellt.

Anschließend wurden die Tabletten in einem Mikrowellenofen für 10 min bei 700 Watt erwärmt. Die Weiterbehandlung erfolgte wie in Beispiel 1 angegeben mit dem Unterschied, dass bei dem Siegelgerät 2 Schienen mit jeweils 5 konkaven Wölbungen verwendet und mit Hilfe von auf 100°C aufgeheizten Siegelbacken jeweils 5 erhitzte Tabletten mit einer Kraft von 1000 N für 30 sek. behandelt wurden.

Die Bruchfestigkeit der Tabletten wird nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

### Beispiel 5:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 7 000 000 (Polyox WSR 303, Dow Chemicals) | 221,0 mg | 132,6 g |
| 100 000 cP (Metholose 90 SH 100 000) | 20,0 mg | 12,0 g |
| Magnesium-Stearat | 9,0 mg | 5,4 g |
| Gesamtgewicht | 350,0 mg | 210,0 g |

Wie in Beispiel 1 angegeben, wurden Tabletten hergestellt.

Anschließend wurden die Tabletten in einem Umluftschrank für 45 min bei 110°C unter N₂-Atmosphäre erwärmt. Die Weiterbehandlung der Tabletten erfolgte wie in Beispiel 4 angegeben mit dem Unterschied, dass die Siegelbacken auf 130°C aufgeheizt wurden.

Die Bruchfestigkeit der Tabletten wird nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

### Beispiel 6:

| Komponenten | Pro Tablette | Gesamtansatz |
|---|---|---|
| Tramadol HCL | 100,0 mg | 60,0 g |
| Polyethylenoxid, NF, MFI (190°C bei 21,6 kg / 10 min) MG 7 000 000 (Polyox WSR 303, Dow Chemicals) | 221,0 mg | 132,6 g |
| Hydroxypropylmethylcellulose 100 000 cP (Metholose 90 SH 100 000) | 20,0 mg | 12,0 g |
| Magnesium-Stearat | 9,0 mg | 5,4 g |
| Gesamtgewicht | 350,0 mg | 210,0 g |

Wie in Beispiel 1 angegeben, wurden Tabletten hergestellt.

Deren Weiterbehandlung erfolgte wie in Beispiel 1 angegeben mit dem Unterschied, dass die Siegelbacken auf 130°C aufgeheizt und die Tabletten in der Unterschiene für 2 Minuten vorerwärmt und währenddessen mit einer Kraft von 10 N belastet wurden. Anschließend wurden die Tabletten mit einer Kraft von 1000 N bei 130°C für 20 sek. nachverdichtet.

Die Bruchfestigkeit der Tabletten wird nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

### Beispiel 7:

| Komponenten | Pro Tablette | Gesamtansatz |
|---|---|---|
| Tramadol HCL | 100 mg | 18,2 g |
| Polyethylenoxid, NF, MG 7 000 000 (Polyox WSR 303 FP, Dow Chemicals) | 165 mg | 30,0 g |
| Polyethylenglykol 6000 | 7 mg | 1,3 g |
| Butylhydroxytoluol | 0,3 mg | 0,1 g |
| Magnesiumstearat | 2,7 mg | 0,5 g |
| Gesamtgewicht | 274,8 mg | 50,0 g |

Die angegebene Butylhydroxytoluol-Menge wurde in 0,6 g Ethanol (96%) gelöst. Diese Lösung wurde mit dem Polyethylenglykol 6000 gemischt und anschließend bei 40°C für 12 h getrocknet. Alle weiteren Komponenten bis auf Magnesiumstearat wurden zugesetzt und in einem Freifallmischer für 15 Minuten gemischt. Anschließend wurde das Magnesiumstearat zugemischt. Die Mischung wurde mit einem 0,8 mm Sieb gesiebt.

Aus der gesiebten Mischung wurden auf einer Ekzenterpresse Korsch Typ EK0 Tabletten hergestellt (Durchmesser:10 mm und Wölbungsradius: 8 mm). Diese wurden anschließend für 15 Minuten in einem Trockenschrank auf 80°C unter N₂-Atmosphäre erwärmt.

Die heißen Tabletten wurden auf einer Ekzenterpresse (Fa. Kilian / IMA Typ: SP 300) mit einer Kraft von 80 kN erneut gepresst. Als Werkzeug wurden Tablettenstempel mit einem Durchmesser von 11 mm und einem Wölbungsradius von 8 mm verwendet.

Die Bruchfestigkeit dieser Tabletten wurde nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37°C und die Umdrehungsgeschwindigkeit des Rührers 75min. Als Freisetzungsmedium wurde Darmsaft mit einem pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 17 % |
| 240 min | 65 % |
| 480 min | 87 % |
| 720 min | 95 % |

## Patentansprüche

1. Verfahren zur Herstellung von festen, pharmazeutischen Darreichungsformen mit zumindest vermindertem Missbrauchspotential, **dadurch gekennzeichnet, dass** man
a) eine Formulierungsmischung enthaltend wenigstens einen Wirkstoff mit Missbrauchspotential, wenigstens ein synthetisches oder natürliches Polymer (C), das eine Bruchfestigkeit von mindestens 500 N aufweist, und ggf. Hilfsstoffe (B) unter einer Krafteinwirkung zu Formlingen formt,
b) gegebenenfalls die Formlinge vereinzelt und gegebenenfalls jeweils nach Größen separiert und
c) nach oder während einer Erwärmung bis wenigstens zum Erweichungspunkt des Polymeren (C) solange unter einer Krafteinwirkung lässt, bis die Formlinge eine Bruchhärte von mindestens 500 N aufweisen, gegebenenfalls mit einer Umhüllung versieht und die Formlinge gegebenenfalls alle wieder vermischt.

2. Verfahren nach Anspruch 1, **dadurch gekennzeichnet, dass** es kontinuierlich oder diskontinuierlich durchgeführt wird.

3. Verfahren nach Anspruch 1 oder 2, **dadurch gekennzeichnet, dass** die Formulierungsmischung mindestens zu 30 Gew.% aus der Komponente (C) besteht.

4. Verfahren nach einem der Ansprüche 1 bis 3, **dadurch gekennzeichnet, dass** die Formulierungsmischung zumindest aus 50 Gew.% aus der Komponente (C) besteht.

5. Verfahren nach einem der Ansprüche 1 bis 4, **dadurch gekennzeichnet, dass** a) die Formgebung der Formulierungsmischung unter einer Krafteinwirkung von mindestens 0,5 kN und gegebenenfalls einer Erwärmung auf weniger 60°C erfolgt.

6. Verfahren nach einem der Ansprüche 1 bis 5, **dadurch gekennzeichnet, dass** gemäß c) die Formlinge vor oder während einer Krafteinwirkung von mindestens 0,1 kN, vorzugsweise von 1 kN bis 120 kN, auf mindestens 60°C erwärmt werden..

7. Verfahren nach einem der Ansprüche 1 bis 6, **dadurch gekennzeichnet, dass** die Krafteinwirkung gemäß a) bzw. c) mit Hilfe einer Presse, vorzugsweise einer Tablettenpresse, Formwalzen oder mit Walzen ausgerüsteten Formbänder ausgeübt wird.

8. Verfahren nach einem der Ansprüche 1 bis 7, **dadurch gekennzeichnet, dass** die Formgebung nach a) zu Tabletten erfolgt.

9. Verfahren nach einem der Ansprüche 1 bis 7, **dadurch gekennzeichnet, dass** die Formgebung nach a) zu einer multipartikulären Darreichungsform mit einer Mindestgröße von 0,5 mm, vorzugsweise von 1 bis 3,5 mm, erfolgt.

10. Verfahren nach einem Ansprüche 1 bis 9, **dadurch gekennzeichnet, dass** als Wirkstoffe opioide Wirkstoffe eingesetzt werden.

## Claims

1. A process for the production of a solid pharmaceutical dosage forms with at least reduced potential for abuse, **characterised in that**
a) a formulation mixture containing at least one active ingredient with potential for abuse, at least one synthetic or natural polymer (C), which exhibits a breaking strength of at least 500 N, and optionally auxiliary substances (B) is shaped into formed articles by application of force,
b) the formed articles are optionally singulated and optionally in each case graded by size and,
c) after or during heating at least to the softening point of the polymer (C), the formed articles are exposed to force until they have a breaking hardness of at least 500 N, they are optionally provided with a cover and all the formed articles are optionally mixed back together again.

2. A process according to claim 1, **characterised in that** it is performed continuously or discontinuously.

3. A process according to claim 1 or claim 2, **characterised in that** the formulation mixture consists to an extent of at least 30 wt.% of component (C).

4. A process according to any one of claims 1 to 3, **characterised in that** the formulation mixture consists to an extent of at least 50 wt.% of component (C).

5. A method according to any one of claims 1 to 4, **characterised in that** a) shaping of the formulation mixture proceeds with application of a force of at least 0.5 kN and optionally with heating to less than 60°C.

6. A process according to any one of claims 1 to 5, **characterised in that**, according to c), the formed articles are heated to at least 60°C before or during application of force of at least 0.1 kN, preferably of 1 kN to 120 kN.

7. A process according to any one of claims 1 to 6, **characterised in that** the application of force according to a) or c) is performed with the assistance of a press, preferably a tablet press, shaping rollers or with shaping belts equipped with rollers.

8. A process according to any one of claims 1 to 7, **characterised in that** shaping according to a) gives rise to tablets.

9. A process according to any one of claims 1 to 7, **characterised in that** shaping according to a) gives rise to a multiparticulate dosage form with a minimum size of 0.5 mm, preferably of 1 to 3.5 mm.

10. A process according to any one of claims 1 to 9, **characterised in that** opioid active ingredients are used as active ingredients.

## Revendications

1. Procédé pour la préparation de formes d'administration pharmaceutiques solides présentant au moins un potentiel d'abus réduit, **caractérisé en ce qu'**on
a) façonne un mélange de formulation contenant au moins une substance active présentant un potentiel d'abus, au moins un polymère synthétique ou naturel (C), qui présente une résistance à la rupture d'au moins 500 N, et le cas échéant un adjuvant (B), sous l'effet d'une force en pièces façonnées,
b) individualise le cas échéant les pièces façonnées et les sépare le cas échéant en fonction des tailles et
c) les laisse sous l'effet d'une force après ou pendant un chauffage au moins jusqu'au point de ramollissement du polymère (C) jusqu'à ce que les pièces façonnées présentent une résistance à la rupture d'au moins 500 N, on les pourvoit le cas échéant d'un enrobage et on mélange le cas échant à nouveau toutes les pièces façonnées.

2. Procédé selon la revendication 1, **caractérisé en ce qu'**il est réalisé de manière continue ou discontinue.

3. Procédé selon la revendication 1 ou 2, **caractérisé en ce que** le mélange de formulation est constitué à raison d'au moins 30% en poids du composant (C).

4. Procédé selon l'une quelconque des revendications 1 à 3, **caractérisé en ce que** le mélange de formulation est constitué à raison d'au moins 50% en poids du composant (C).

5. Procédé selon l'une quelconque des revendications 1 à 4, **caractérisé en ce que** le façonnage a) du mélange de formulation est réalisé sous l'effet d'une force d'au moins 0,5 kN et le cas échéant d'un chauffage à moins de 60°C.

6. Procédé selon l'une quelconque des revendications 1 à 5, **caractérisé en ce que** selon c) les pièces façonnées sont chauffées avant ou pendant l'effet d'une force d'au moins 0,1 kN, de préférence de 1 kN à 120 kN, à au moins 60°C.

7. Procédé selon l'une quelconque des revendications 1 à 6, **caractérisé en ce que** l'effet d'une force selon a) ou c) est réalisé à l'aide d'une presse, de préférence d'une presse à comprimés, des cylindres de façonnage ou des bandes de façonnage équipées de cylindres.

8. Procédé selon l'une quelconque des revendications 1 à 7, **caractérisé en ce que** le façonnage selon a) est réalisé en comprimés.

9. Procédé selon l'une quelconque des revendications 1 à 7, **caractérisé en ce que** le façonnage selon a) est réalisé en une forme d'administration multiparticulaire présentant une grosseur minimale de 0,5 mm, de préférence de 1 à 3,5 mm.

10. Procédé selon l'une quelconque des revendications 1 à 9, **caractérisé en ce qu'**on utilise comme substances actives des substances actives opioïdes.
